Europäisches Patentamt

(19) European Patent Office

Office européen des brevets

(11) Publication number: **0 086 290**
**B1**

(12) EUROPEAN PATENT SPECIFICATION

(45) Date of publication of patent specification: **29.01.86**

(51) Int. Cl.⁴: **A 61 K 7/16**

(21) Application number: **82300730.7**

(22) Date of filing: **15.02.82**

(54) **Dental hygiene compositions and their production.**

(43) Date of publication of application:
**24.08.83 Bulletin 83/34**

(45) Publication of the grant of the patent:
**29.01.86 Bulletin 86/05**

(84) Designated Contracting States:
**AT BE CH DE FR GB IT LI LU NL SE**

(56) References cited:
**EP-A-0 002 594**
**US-A-2 054 742**
**US-A-4 238 476**
**US-A-4 301 143**

**H. JANISTYN: "Handbuch der Kosmetika und Riechstoffe", vol. 1, Die kosmetischen Grundstoffe, A. Hüthig Verlag, Heidelberg. DE, 3rd edition 1978, pages 925-926**
**ZAHNÄRZTLICHE WELT, ZAHNÄRZTLICHE RUNDSCHAU, ZAHNÄRZTLICHE REFORM, vol. 82, no. 7, 1973, pages 328-331, A. Hüthig Verlag, Heidelberg, DE. P. RIETHE: "Die Hemmung unverkalkter und verkalter Ablagerungen durch Na-Laurylsulfat und Na-Sulforicinoleat"**

(73) Proprietor: **WARNER-LAMBERT COMPANY**
**201 Tabor Road**
**Morris Plains New Jersey 07950 (US)**

(72) Inventor: **Ambike, Suhas Hanumant**
**715 Military Trail**
**Westhill Ontario MIE 4P6 (CA)**
Inventor: **Blaser, Eric**
**36 Austin Terrace**
**Toronto Ontario M5R 1Y5 (CA)**
Inventor: **Grewal, Narinder Singh**
**64 Dundalk Drive**
**Scarborough Ontario MIP 4T7 (CA)**

(74) Representative: **Jones, Michael Raymond et al**
**HASELTINE LAKE & CO. Hazlitt House 28**
**Southampton Buildings Chancery Lane**
**London WC2A 1AT (GB)**

## Description

This invention relates to dental hygiene compositions including, but not limited to, dentifrices in solid, powder, paste, or cream form and liquid mouth washes and rinses, and to the production of such compositions.

The primary use of such compositions is in maintaining dental hygiene and it is the function of such compositions to keep the surface of the teeth as clean and shiny as possible, to preserve the health of the teeth and gums, to inhibit the formation of unpleasant odours in the oral cavity and to freshen the user's breath. Known formulations of such compositions have been intended to provide cosmetic, cleansing, refreshing and/or deodorizing benefits, or have provided a therapeutic activity, e.g. anticaries activity.

The compositions of the present invention are formulated to provide a further therapeutic activity, namely an antiplaque activity. Dental plaque is considered to be a material composed largely of microorganisms and an organic matrix derived from bacteria and saliva. Dental experts generally consider that the calculus also known as tartar is a mature plaque which has crystallized with the development of an indentifiable crystal structure. It is well known that even with regular and thorough brushing, the plaque (i.e. calcified plaque) deposit adheres tenaciously to the teeth. Such deposits are unattractive and cause tooth decay.

According to one aspect of the present invention, there is provided a dental hygiene composition having antimicrobial activity against plaque-forming microorganisms and comprising an effective amount of from 0.1 to 2.0% by weight, based on the weight of the composition, of one or more highly pure alkali metal salts of dodecyl sulphate having a content of less than 5% of non-dodecyl alkyl sulphate salt(s), 0.5 to 2.0% by weight, based on the weight of the composition, of an alkali metal N-acyl sarcosinate, and a physiologically-acceptable acidic buffer for maintaining the pH of the composition in the range from 3.0 to 5.0, contained in one or more vehicles physiologically compatible with human teeth and mouth tissues.

According to another aspect of the present invention, there is provided a process for producing a dental hygiene composition having antimicrobial activity against plaque-forming microorganisms, which process comprises incorporating an effective amount of from 0.1 to 2.0% by weight, based on the weight of the composition, of one or more highly pure alkali metal salts of dodecyl sulphate having a content of less than 5% of non-dodecyl alkyl sulphate salt(s), 0.5 to 2.0% by weight of an alkali metal N-acyl sarcosinate, and a physiologically-acceptable acidic buffer for maintaining the pH of the composition in the range from 3.0 to 5.0, in one or more vehicles physiologically compatible with human teeth and mouth tissues.

The compositions of the present invention are intended to prevent or reduce the deposit of dental plaque. The compositions of the present invention may also contain anticaries agent such as sodium fluoride or sodium monofluorophosphate.

The present invention is based in part on the finding that alkali metal salts of dodecyl (lauryl) sulphate when employed in a highly pure form, with a content of less than 5% of any other alkyl sulphate, exhibit particular advantages when incorporated in dental hygiene compositions having antiplaque activity. The most readily-available lauryl sulphate salt is the sodium form, i.e. sodium lauryl sulphate. The potassium and other alkali metal salts are analogous to the sodium salt, although the use of the latter is normally preferred for reasons of economy. It will be appreciated, however, that the potassium and other alkali metal salts may be substituted for the sodium salt in the compositions of the present invention hereinafter described.

The material that is commonly commercially available under the designation "sodium lauryl sulphate" is not the pure compound sodium dodecyl sulphate $CH_3(CH_2)_{10}CH_2OSO_3Na$, but is a mixture of homologous sodium alkyl sulphates with sodium dodecyl sulphate predominating. Thus, as normally employed, the designation or chemical description "sodium lauryl sulphate" refers to a material containing as impurity significant and varying amounts of sodium salts of non-dodecyl alkyl sulphates as well as the pure dodecyl sulphate salt. These commercially available materials often also contain varying amounts of sodium salts of inorganic acids, e.g. sodium chloride and sodium sulphate, and unsulphated alcohols.

Commercially available sodium lauryl sulphate in impure form has been employed in the past in toothpaste compositions as a detergent or foam-producing agent. An investigation has been made of the specific antimicrobial activity of solutions of various forms of material commercially available as "sodium lauryl sulphate", including one form that has recently become available from certain sources as a highly pure compound substantially free from any content of non-dodecyl alkyl sulphate sodium salts. It has been found that the antimicrobial activity of the pure form of the sodium dodecyl sulphate material against the microorganisms considered to be responsible for plaque-formation in the human mouth is high, while the activity of the impure solutions combining substantial amounts of non-dodecyl sulphates against such microorganisms is inhibited to a significant extent.

Thus as between pure and impure solutions containing equivalent amounts of the pure compound, a higher specific activity was found to be exhibited by the solution that was substantially free of non-dodecyl alkyl sulphate salts, and therefore, by employing sodium dodecyl sulphate material in a form that is of high purity, there can be provided a composition having an effective level of antiplaque activity at a relatively lower content of the alkyl sulphate salt. The level of the alkyl sulphate is a matter of practical importance as the concentration of alkyl sulphate that needs to be employed in a mouthwash or toothpaste composition can be an important factor in the formation of a product that meets with consumer acceptance. In the mouth, sodium dodecyl sulphate and other alkyl sulphates have an astringent effect. Depending on the nature and proportions of the ingredients in the formulation, a small variation in the alkyl sulphate content can make the difference between a product that is acceptable to the consumer and one that is unacceptable as having a harsh and unpleasant taste or that gives an unpleasant sensation in the mouth owing to the physiological action of the alkyl sulphate on the mouth and dental tissues. Moreover, an excessive content of alkyl sulphate is normally to be avoided as in use of the composition this can lead to an overabundant degree of foaming in the mouth.

In the dental hygiene composition of the present invention, an effective amount of from 0.1 to 2.0 percent of one or more highly pure alkali metal salts of dodecyl sulphate substantially free from non-dodecyl alkyl sulphate salts, is contained, preferably dissolved, in one or more vehicles physiologically compatible with the teeth and mouth tissues.

All parts and percentages herein are by weight unless specified otherwise.

Any suitably pure form of sodium dodecyl sulphate or other alkli metal dodecyl sulphate salt may be employed in the compositions of the present invention. The amount of non-dodecyl impurity that can be tolerated in the composition without significantly detracting from the antiplaque activity depends in each case on the quantity of the pure sodium or other alkali metal dodecyl sulphate that is present, and the percentages of impurity are therefore best expressed in terms of the weight of alkali metal dodecyl sulphate. The compositions of the present invention contain less than 5% of salts of non-dodecyl alkyl sulphate, based on the weight of alkali metal dodecyl sulphate, preferably less than 2% and more preferably less than 1% based on the weight of the alkali metal dodecyl sulphate salt. One especially preferred source of the alkali metal dodecyl sulphate salt for use in the composition of the invention is the highly pure form of sodium dodecyl sulphate that is available under the trade mark TEXAPON L-100 from Henkel Chemicals (Canada) Ltd., of Toronto, Canada. This material has a washing activity of 99%, indicating a content of pure sodium dodecyl sulphate of about 99%, and contains less than 0.6% of alkali metal chloride and sulphate and has a zero content of unsulphated alcohols. It is expected that the TEXAPON L-100 material is unique among other commercially-available forms of "sodium lauryl sulphate" in its high content of the pure dodecyl sulphate salt, but it will be appreciated that the invention is not necessarily limited to the use of the TEXAPON L-100 material and that such other highly pure forms of the dodecyl sulphate salt as may be available, or may in the future become available, having a similar high degree of purity of at least 95%, may be employed.

It should be noted that preferred compositions of the present invention are substantially free from any content of fatty acid-based soaps or detergents other than the alkali metal dodecyl sulphate salts, as these may impart an undesirable taste without contributing to the antiplaque activity of the pure dodecyl sulphate salt, and therefore the desirable antiplaque activity of the compositions is exhibited to the best advantage when solutions free from these materials were employed.

The antimicrobial activity of the compositions of the present invention varies with pH and the optimum activity of the dodecyl sulphate salts has been found to occur when the salts are acidulated to about pH 2. However, under extremely acidic conditions, the alkali metal dodecyl sulphate salt tends to undergo hydrolysis so that the antiplaque activity diminishes over prolonged periods, e.g. during storage. At a pH in the range of 3.0 to 5.0 satisfactory antiplaque activity can usually be achieved without undue loss of activity occurring during storage of the made-up compositions. Optimum antiplaque activity extending over normal storage life can be obtained at about pH 4.5 and this pH is therefore most preferred for present purposes. However, in order to achieve this pH depending on the basicity of other ingredients of the composition, it may be necessary to add considerable quantities of aqueous solutions of acid, and in some formulations, particularly in the case of toothpastes and like solid or semi-solid dentifrices containing abrasives or other materials that exhibit a buffering action tending to maintain a pH higher than 4.5, the amount of liquid that can be added is limited owing to the requirement for a viscous consistency. In such cases pH somewhat higher than 4.5 will normally be employed, i.e. pH 4.5 to 5.0 consistent with maintaining a desired solids content in the composition. Any physiologically acceptable acid may be employed to adjust the pH to the desired value. Suitable examples include dilute hydrochloric acid and phosphoric acid. Physiologically acceptable acidic buffers, e.g. buffering salt pairs such as the well-known acetate or citrate buffers i.e. sodium acetate-acetic acid buffer or sodium tricitrate/citric acid buffer, or buffer compounds such as dibasic sodium phosphate may be added, typically in an amount of from 0.01 to 0.2%, to maintain the compositions at the desired pH during mixing and storage.

Certain types of liquid dental hygiene formulations, especially mouthwashes and rinses, conventionally contain substantial quantities of ethyl alcohol as an astringent and antiseptic agent. It has been found that in the presence of substantial quantities of ethyl alcohol the desired antiplaque activity can be achieved with a reduced content of the pure alkali metal dodecyl sulphate salts, and it is suggested that the presence of ethyl alcohol potentiates the antiplaque activity of the dodecyl sulphate salts. Accordingly, in liquid mouthwash compositions containing substantial quantities of ethyl alcohol, in the range from 10 to 30%, a somewhat smaller quantity of the alkali metal dodecyl sulphate salt may be employed, preferably in the range from 0.1 to 0.5%.

The compositions of the present invention may be flavoured with conventional essential oil flavours, e.g. thymol, eucalyptol, methyl salicylate, and peppermint and spearmint oils, typically in an amount of from 0.2 to 3.0%. In the case of compositions combining water or another aqueous vehicle, these oils may if necessary be maintained in dispersion with the aid of suitable dispersing agents. The use of cationic detergents is to be avoided for this purpose as these materials tend to reduce the efficacy of the alkali metal dodecyl sulphate salts and the preferred dispersing agents are nonionic surfactants advantageously in an amount of from 0.1 to 0.2%. As an example of a nonionic surfactant, there may be mentioned the water-soluble TWEEN (trade mark) materials, which are polyoxyethylene derivatives of fatty acid partial esters of sorbitol anhydrides, but these tend to have a pronounced taste that may not be desired in all instances and therefore the preferred nonionic surfactants are the water-soluble PLURONIC (trade mark) material available from BASF Wyandotte Corporation. These are alpha-hydro omega-hydroxy polyoxyethylene block copolymers. Especially preferred on account of its excellent dispersing and solubilizing activity and because it is relatively tasteless, is the PLURONIC F-127 nonionic surfactant which is available as a white solid, of average molecular weight 12,500, in prill or flake form.

In the case of compositions in accordance with the present invention that are formulated as liquid mouthwashes or rinses, it is further preferred to add zinc chloride as an astringent, suitably in an amount of from 0.01 to 0.05%. This material has mouth deodorizing activity and in the presence of sodium dodecyl sulphate this activity is potentiated.

It has been further found that compositions containing a mixture of the pure alkali metal dodecyl sulphate together with alkali metal N-acyl sarcosinates exert a greater antiplaque activity than compositions containing equivalent weights of either of these compounds without the other, or that, in other words, the combination of the dodecyl sulphate salt with the N-acyl sarcosinate achieves a synergistic effect. For example, in comparisions of the antiplaque activity of five toothpaste compositions that contained 2% alkali metal dodecyl sulphate, 2% alkali metal N-acyl sarcosinate, and 2% of one of three mixtures of the said sulphate and sarcosinate in the ratios 1:1, 1:2, and 2:1, respectively, there were observed percentage reductions of plaque of 50%, 40%, 78%, 61% and 60% respectively. This action of the N-acyl sarcosinates in potentiating the activity of the alkali metal dodecyl sulphates may be exploited in the formulation of toothpaste and like dentrifrices in solid, powder, paste, or cream form, where a composition of high solids content with an enhanced antiplaque activity may be achieved at a relatively low content of the alkali metal dodecyl sulphate through inclusion of the N-acyl sarcosinate, in the formulation. The preferred material is sodium N-lauroyl sarcosinate added in an amount of from 0.5 to 2.0% by weight, more preferably in 1:1 to 1:3 ratio to the weight of alkali metal dodecyl sulphate, the latter preferably also being present in an amount of 0.5 to 2.0% by weight.

The composition may contain the conventional humectants such as glycerin and/or sorbitol. In the case of mouthwashes containing relatively large quantities of water, e.g. in the range 50 to 90%, the humectants may be present in an amount up to 20%, more typically about 10%. In the case of toothpaste and like products where the moisture content plays a more important role in preserving a desired consistency and viscosity of the product, somewhat greater quantities of humectants, e.g. in the range from 20 to 40%, may be used.

Typically, toothpaste formulations in accordance with the present invention will contain from 25 to 40% particulate dental abrasive, from 1 to 4% thickener such as carboxymethyl cellulose derivatives and/or polyethylene glycols, together with the humectant, dodecyl salt, and N-acyl sarcosinate salt, and may also contain a physiologically acceptable pigment, an artificial sweetener agent, e.g. sodium saccharin or sodium cyclamate, an acid and buffer to maintain a desired pH, and an essential oil flavour.

Both the liquid and the solid or semi-solid e.g. paste, formulations in accordance with the present invention may contain small amounts of conventional fluoride anticarries agents. In the case of liquid mouthwashes or rinses this may suitably comprise sodium fluoride in an amount of from 0.01 to 0.05% and in the case of toothpaste the preferred material is sodium monofluorophosphate in an amount of from 0.5 to 2.0%, although other anticaries fluoride e.g. stannous fluoride or sodium fluoride may also be used.

A preferred liquid mouthwash comprises from 0.1 to 0.5% of highly pure sodium dodecyl sulphate, from 0.5 to 2.0% by weight of an alkali metal N-acyl sarcosinate, from 10 to 30% ethyl alcohol, from 0.01 to 0.05% zinc chloride, from 0 to 20% humectant, from 0.2 to 3.0% essential oil flavour, from 0.1 to 0.2 non-ionic dispersing agent, from 0.002 to 0.005% dye, physiologically acceptable acid in amount sufficient to bring the pH of the composition to a pH in the range from 3.0 to 5.0, buffer solution for maintaining the pH of the composition at a pH in the range from 3.0 to 5.0, from 0.01 to 0.05% fluoride anticaries agent and the balance water, all percentages being by the weight.

A preferred toothpaste composition comprises an effective amount of from 0.5 to 2.0% of one or more highly pure alkali metal salts of dodecyl sulphate substantially free from non-dodecyl alkyl sulphate salt contained in one or more vehicles physiologically compatible with human teeth and mouth tissues, from 0.5 to 2.0% of alkali metal N-acyl sarcosinate, from 25 to 40% particulate dental abrasive, from 1 to 4% thickener, and from 20 to 40% humectant, the balance comprising water, and all percentages being by weight.

A more preferred toothpaste composition comprises from 0.5 to 2.0% highly pure sodium dodecyl sulphate, from 25 to 40% particulate dental abrasive, from 1 to 4% thickener, from 0.5 to 3.0% physiologically-acceptable pigment, from 0.5 to 2.0% sodium N-lauroyl sarcosinate, from 20 to 40% humectant, from 0.1 to 0.3% sweetener, from 0.5 to 1% fluoride anticaries agent, from 1.0 to 5.0% phosphate buffer, sufficient phosphoric acid to adjust the pH of the composition to about pH 5.0, approximately 0.5% essential oil flavour, the balance being water, and all percentages being by weight.

Although the above description provides ample information for those skilled in the art to formulate dental hygiene compositions in accordance with the present invention having antiplaque activity, for further illustration certain specific formulations will be given by way of example only, together with a test method for determining antiplaque activity.

Example 1

The ingredients listed in the following Table 1 were blended together in the percentages by weight indicated to yield a smooth paste.

Ranges of preferred percentages by weight are also indicated.

TABLE 1

| Ingredients | Percent | Preferred Range—Percent |
|---|---|---|
| Precipitated silicon dioxide** | 32.0 | 25—40 |
| Sodium carboxymethyl cellulose (thickener) | 1.0 | 0.5—2.0 |
| Polyethylene glycol 4000 (thickener) | 1.0 | 0.5—2.0 |
| Titanium dioxide (pigment) | 1.0 | 0.5—3.0 |
| Texapon L-100* | 0.5 | 0.5—2.0 |
| Sodium N-lauryl sarcosinate | 1.0 | 0.5—2.0 |
| Glycerine (humectant) | 16.0 | 10.0—20 |
| Sorbitol (humectant) | 16.0 | 10.0—20 |
| Artificial sweetener (sodium saccharin or cyclamate) | 0.2 | 0.1—0.3 |
| Sodium monofluoro phosphate | 0.7 | 0.5—1.0 |
| Sodium phosphate dibasic (phosphate buffer) | 1.0 | 1.0—5.0 |
| Phosphoric acid | q.s to adjust to pH 5 | q.s. to adjust to pH 3—6 |
| Essential oil flavour | 1.1 | 0.5—5 |
| Water | Balance | 20—40 |

* (trade mark) high purity sodium dodecyl sulphate available from Henkel Chemicals (Canada) Ltd.
**Precipitated silicon dioxide available from J. M. Huber Corporation, Harve de Grace, Maryland, U.S.A. under the trade mark ZEODENT 113, having a particle size from 100—300 angstrom units and a surface area from 10 m²/g to 300 m²/g.

Test method

The antimicrobial activity of solutions of sodium lauryl sulphate can be determined as follows:

Tooth units are prepared in the form of 24×10×3 mm acrylic plastic tiles.

A medium is prepared comprising Trypticase soy broth obtained from Baltimore Biological Laboratories with 5% added sucrose sterilized by autoclaving in 500 ml quantities. The 500 ml quantities are then inoculated with a standard quantity of plaque-forming microorganisms. In successive tests, the inoculum consists of 0.5 ml from human saliva, and bacterial cultures of *S. aureus, St. faecalic, Ps. aeruginosa, E. coli, C. albicans, S. cerevisiae,* and *A. viscosus,* each containing uniform number of cells, suitably of the order of $5 \times 10^8$ cells per inoculum. After inoculation, 10 ml aliquots are aseptically transferred to sterile 150×20 mm test tubes containing the plastic tiles. The tubes are incubated at 37°C for 24 hours. The tubes are then shaken on a Vortex Mixer for 10 seconds to remove loose pellicle which is allowed to settle. The tiles are then transferred to test tubes containing the test solutions for 30 seconds and are then put into tubes containing sterile distilled water for the same period. Control tiles receive two 30 seconds treatment with sterile water. The tiles are then replaced into the test tubes containing inoculated medium broth and are incubated at 37°C. After 3 treatments, the plaque adhering on the tiles is measured employing a conventional chemical protein-determination procedure.

**Claims for the Contracting States: BE, CH, DE, FR, GB, IT, LI, LU, NL, SE**

1. A dental hygiene composition having antimicrobial activity against plaque-forming micro-organisms and comprising an effective amount of from 0.1 to 2.0% by weight based on the weight of the composition, of one or more highly pure alkali metal salts of dodecyl sulphate having a content of less than 5% of non-dodecyl alkyl sulphate salt(s), 0.5 to 2.0% by weight, based on the weight of the composition, of an alkali metal N-acyl sarcosinate, and a physiologically-acceptable acidic buffer for maintaining the pH of the composition in the range from 3.0 to 5.0, contained in one or more vehicles physiologically compatible with human teeth and mouth tissues.

2. A composition as claimed in claim 1, wherein said content of non-dodecyl alkyl sulphate salt(s) is less than 2%.

3. A composition as claimed in claim 1, wherein said content is of non-dodecyl alkyl sulphate salt(s) is less than 1%.

4. A composition as claimed in any preceding claim, wherein the vehicle comprises water and the pH of the composition is approximately 4.5.

5. A composition as claimed in any one of claims 1 to 4, which contains from 0.5 to 2.0% by weight of said highly pure alkali metal salt of dodecyl sulphate.

6. A liquid mouthwash composition having antimicrobial activity against plaque-forming microorganisms and comprising from 0.1 to 2.0% by weight of one or more highly pure alkali metal salts of dodecyl sulphate having a content of less than 5% of non-dodecyl alkyl sulphate salt(s), 0.5 to 2.0% by weight of an alkali metal N-acyl sarcosinate, and a physiologically-acceptable acidic buffer for maintaining the pH of the composition in the range from 3.0 to 5.0, dissolved in one or more vehicles physiologically compatible with human teeth and mouth tissues, and from 10 to 30% by weight of ethyl alcohol, the percentages being with respect to the weight of the composition.

7. A liquid mouthwash composition as claimed in claim 6, which contains from 0.1 to 0.5% by weight of the highly pure alkali metal salt of dodecyl sulphate.

8. A liquid mouthwash composition as claimed in claim 6 or 7, which contains water or another aqueous vehicle, and also contains from 0.2 to 3.0% by weight of essential oil flavour and from 0.1 to 0.2% by weight of non-ionic surfactant(s) as dispersing agent therefor.

9. A liquid mouthwash composition as claimed in claim 6, 7 or 8, which has a water content of from 50 to 90% by weight and contains up to 20% by weight of humectant.

10. A liquid mouthwash composition as claimed in any one of claims 6 to 9, which also contains from 0.01 to 0.05% by weight of zinc chloride dissolved in water or another aqueous medium as the vehicle.

11. A liquid mouthwash composition as claimed in claim 6 and comprising from 0.1 to 0.5% of highly pure sodium dodecyl sulphate, from 0.5 to 2.0% by weight of an alkali metal N-acyl sarcosinate, from 10 to 30% ethyl alcohol, from 0.01 to 0.05% zinc chloride, from 0 to 20% humectant, from 0.2 to 3.0% essential oil flavour, from 0.1 to 0.2 non-ionic dispersing agent, from 0.002 to 0.005% dye, physiologically acceptable acid in an amount sufficient to bring the pH of the composition to a pH in the range from 3.0 to 5.0, buffer solution for maintaining the pH of the composition at a pH in the range from 3.0 to 5.0, from 0.01 to 0.05% fluoride anticaries agent and the balance water, all percentages being by weight.

12. A toothpaste composition having antimicrobial activity against plaque-forming micro-organisms and comprising an effective amount of from 0.5 to 2.0% of one or more highly pure alkali metal salts, of dodecyl sulphate having a content of less than 5% of non-dodecyl alkyl sulphate salt(s) contained in one or more vehicles physiologically compatible with human teeth and mouth tissues, a physiologically-acceptable acidic buffer for maintaining the pH in the range from 3.0 to 5.0, from 0.5 to 2.0% of an alkali metal N-acyl sarcosinate, from 25 to 40% particulate dental abrasive, from 1 to 4% thickener, and from 20 to 40% humectant, the balance comprising water, and all percentages being by weight.

13. A toothpaste composition as claimed in claim 12, which also contains a fluoride anticaries agent.

14. A toothpaste composition as claimed in claim 13, wherein the fluoride is sodium monofluorophosphate.

15. A composition as claimed in any preceding claim, wherein said sarcosinate is N-lauroyl sarcosinate.

16. A composition as claimed in claim 15, wherein said sarcosinate is sodium N-lauroyl sarcosinate.

17. A toothpaste composition as claimed in claim 12, comprising from 0.5 to 2.0% highly pure sodium dodecyl sulphate, from 25 to 40% particulate dental abrasive, from 1 to 4% thickener, from 0.5 to 3.0% physiologically-acceptable pigment, from 0.5 to 2.0% sodium N-lauroyl sarcosinate, from 20 to 40% humectant, from 0.1 to 0.3% sweetener, from 0.1 to 1% fluoride anticaries agent, from 1.0 to 5.0% phosphate buffer, sufficient phosphoric acid to adjust the pH of the composition to about pH 5.0, approximately 0.5% essential oil flavour, the balance being water, and all percentages being by weight.

**Claims for the Contracting State: AT**

1. A process for producing a dental hygiene composition having antimicrobial activity against plaque-forming micro-organisms, which process comprises incorporating an effective amount of from 0.1 to 2.0% by weight, based on the weight of the composition, one or more highly pure alkali metal salts of dodecyl sulphate having a content of less than 5% of non-dodecyl alkyl sulphate salt(s), 0.5 to 2.0% by weight, based on the weight of the composition, of an alkali metal N-acyl sarcosinate, and a physiologically-acceptable acidic buffer for maintaining the pH of the composition in the range from 3.0 to 5.0, in one or more vehicles physiologically compatible with human teeth and mouth tissues.

2. A process according to claim 1, wherein said content of non-dodecyl alkyl sulphate salt(s) is less than 2%.

3. A process according to claim 1, wherein said content of non-dodecyl alkyl sulphate salt(s) is less than 1%.

4. A process according to any preceding claim, wherein the vehicle comprises water and the pH of the composition is approximately 4.5.

5. A process according to any one of claims 1 to 4, wherein the composition contains from 0.5 to 2.0% by weight of said highly pure alkali metal salt of dodecyl sulphate.

6. A process for producing a liquid mouthwash composition having antimicrobial activity against plaque-forming microorganisms, which process comprises incorporating into a suitable base composition from 0.1 to 2.0% by weight of one or more highly pure alkali metal salts of dodecyl sulphate having a content of less than 5% of non-dodecyl alkyl sulphate salt(s), 0.5 to 2.0% by weight of an alkali metal N-acyl sarcosinate and a physiologically acceptable acidic buffer for maintaining the pH of the composition in the range from 3.0 to 5.0, dissolved in one or more vehicles physiologically compatible with human teeth and mouth tissues, and from 10 to 30% by weight of ethyl alcohol, the percentages being with respect to the weight of the final composition.

7. A process according to claim 6, which contains from 0.1 to 0.5% by weight of the highly pure alkali metal salt of dodecyl sulphate.

8. A process according to claim 6 or 7, wherein the composition contains water or another aqueous vehicle, and also contains from 0.2 to 3.0% by weight of essential oil flavour and from 0.1 to 0.2% by weight of non-ionic surfactant(s) as dispersing agent therefor.

9. A process according to claim 6, 7 or 8, wherein the composition has a water content of from 50 to 90% by weight and contains up to 20% by weight of humectant.

10. A process according to any one of claims 6 to 9, wherein the composition contains from 0.01 to 0.05% by weight of zinc chloride dissolved in water or another aqueous medium as the vehicle.

11. A process according to claim 6, which comprises incorporating in water from 0.1 to 0.5% of highly pure sodium dodecyl sulphate, from 0.5 to 2.0% by weight of an alkali metal N-acyl sarcosinate, from 10 to 30% ethyl alcohol, from 0.01 to 0.05% zinc chloride, from 0 to 20% humectant, from 0.2 to 3.0% essential oil flavour, from 0.1 to 0.2 non-ionic dispersing agent, from 0.002 to 0.005% dye, physiologically acceptable acid in an amount sufficient to bring the pH of the composition to a pH in the range from 3.0 to 5.0, buffer solution for maintaining the pH of the composition at a pH in the range from 3.0 to 5.0, and from 0.01 to 0.05% fluoride anticarries agent, all percentage being by weight and being with respect to the composition.

12. A process for producing a toothpaste composition having antimicrobial activity against plaque-forming microorganisms, which comprises incorporating in water an effective amount of from 0.5 to 2.0% of one or more highly pure alkali metal salts of dodecyl sulphate having a content of less than 5% of non-dodecyl alkyl sulphate salt(s) dissolved in one or more vehicles physiologically compatible with human teeth and mouth tissues, a physiologically-acceptable acidic buffer for maintaining the pH in the range from 3.0 to 5.0, from 0.5 to 2.0% of an alkali metal N-acyl sarcosinate, from 25 to 40% particulate dental abrasive, from 1 to 4% thickener, and from 20 to 40% humectant, all percentages being by weight and with respect to the composition.

13. A process according to claim 12, which also comprises incorporating in the composition a fluoride anticarries agent.

14. A process according to claim 13, wherein the fluoride is sodium monofluorophosphate.

15. A process according to any preceding claim, wherein said sarcosinate is N-lauroyl sarcosinate.

16. A process according to claim 15, wherein said sarcosinate is sodium N-lauroyl sarcosinate.

17. A process for producing a toothpaste composition in accordance with claim 12, which comprises incorporating in water from 0.5 to 2.0% highly pure sodium dodecyl sulphate, from 25 to 40% particulate dental abrasive, from 1 to 4% thickener, from 0.5 to 3.0% physiologically-acceptable pigment, from 0.5 to 2.0% sodium N-lauroyl sarcosinate, from 20 to 40% humectant, from 0.1 to 0.3% sweetener, from 0.5 to 1% fluoride anti-caries agent, from 1.0 to 5.0% phosphate buffer, sufficient phosphoric acid to adjust the pH of the composition to about pH 5.0, and approximately 0.5% essential oil flavour, all percentages being by weight and with respect to the composition.

**Patentansprüche für die Vertragsstaaten: BE, CH, DE, FR, GB, IT, LI, LU, NL, SE**

1. Dentalhygienische Zusammensetzung mit antimikrobieller Wirkung gegen Plaque-bildende Mikroorganismen und dadurch gekennzeichnet, daß sie eine wirksame Menge von 0,1 bis 2,0 Gew.-%, bezogen auf das Gewicht der Zusammensetzung, eines oder mehrerer hochreine(n)r Alkalimetallsalze(s) von Dodecylsulfat mit einem Gehalt von weniger als 5% an nicht-Dodecyl Alkylsulfatsalz(en), 0,5 bis 2,0 Gew.-%, bezogen auf das Gewicht der Zusammensetzung eines Alkalimetall-N-acylsarcosinats und einen physiologisch akzeptablen sauren Puffer zur Aufrechterhaltung des pH Wertes der Zusammensetzung im Bereich von 3,0 bis 5,0, welche in einem oder mehreren mit menschlichen Zähnen und Mundgeweben physiologisch verträglichen Träger(n) enthalten sind, umfaßt.

2. Zusammensetzung nach Anspruch 1, dadurch gekennzeichnet, daß der Gehalt an nicht-Dodecyl Alkylsulfatsalz(en) weniger als 2% beträgt.

3. Zusammensetzung nach Anspruch 1, dadurch gekennzeichnet, daß der Gehalt an nicht-Dodecyl Alkylsulfatsalz(en) weniger als 1% beträgt.

4. Zusammensetzung nach irgendeinem der vorhergehenden Ansprüche, dadurch gekennzeichnet, daß der Träger Wasser umfaßt, und der pH Wert der Zusammensetzung ungefähr 4,5 ist.

5. Zusammensetzung nach irgendeinem der Ansprüche 1 bis 4, dadurch gekennzeichnet, daß sie von 0,5 bis 2,0 Gew.-% des hochreinen Alkalimetallsalzes von Dodecylsulfat enthält.

6. Flüssige Mundwasserzusammensetzung mit antimikrobieller Wirkung gegen Plaque-bildende Mikroorganismen und dadurch gekennzeichnet, daß sie von 0,1 bis 2,0 Gew.-% eines oder mehrerer hochreiner Alkalimetallsalze(s) von Dodecylsulfat mit einem Gehalt von weniger als 5% an nicht-Dodecyl Alkylsulfatsalz(en), 0,5 bis 2.0 Gew.-% eines Alkalimetall-N-acrylsarcosinats und eines physiologisch akzeptablen sauren Puffers zur Aufrechterhaltung des pH Wertes des Zusammensetzung im Bereich von 3,0 bis 5,0, welche in einem oder mehreren mit menschlichen Zähnen und Mundgeweben physiologisch verträglichen Träger(n) und in von 10 bis 30 Gew.-% Äthylalkohol gelöst sind, wobei die Prozentsätze sich auf das Gewicht der Zusammensetzung beziehen, umfaßt.

7. Flüssige Mundwasserzusammensetzung nach Anspruch 6, dadurch gekennzeichnet, daß sie von 0,1 bis 0,5 Gew.-% des hochreinen Alkalimetallsalzes von Dodecylsulfat enthält.

8. Flüssige Mundwasserzusammensetzung nach den Ansprüche 6 oder 7, dadurch gekennzeichnet, daß sie Wasser oder einen anderen wässrigen Träger enthält und auch von 0,2 bis 3,0 Gew.-% ätherisches Öl als Geschmacksstoff und von 0,1 bis 0,2 Gew.-% nicht-ionische(s) oberflächenaktive(s) Mittel als Dispergierungsmittel dafür enthält.

9. Flüssige Mundwasserzusammensetzung nach den Ansprüchen 6, 7 oder 8, dadurch gekennzeichnet, daß sie einen Wassergehalt von 50 bis 90 Gew.-% hat und bis zu 20 Gew.-% Feuchthaltemittel enthält.

10. Flüssige Mundwasserzusammensetzung nach irgendeinem der Ansprüche 6 bis 9, dadurch gekennzeichnet, daß sie auch von 0,01 bis 0,05 Gew.-% von in Wasser oder einem anderen wässrigen Medium als Träger gelösten Zinkchlorid enthält.

11. Flüssige Mundwasserzusammensetzung nach Anspruch 6 und dadurch gekennzeichnet, daß sie von 0,1 bis 0,5% hochreines Dodecylsulfat, von 0,5 bis 2,0 Gew.-% eines Alkalimetall-N-acylsarcosinats, von 10 bis 30% Äthylalkohol, von 0,01 bis 0,05% Zinkchlorid, von 0 bis 20% Feuchthaltemittel, von 0,3 bis 3,0% ätherisches Öl als Geschmacksstoff, von 0,12 bis 0,2% nicht-ionisches Dispergierungsmittel, von 0,002 bis 0,005% Farbstoff, eine ausreichende Menge an physiologisch akzeptabler Säure, um den pH Wert der Zusammensetzung in den Bereich von 3,0 bis 5,0 du bringen, Pufferlösung zur Aufrechterhaltung des pH Wertes der Zusammensetzung bei einem pH Wert im Bereich von 3,0 bis 5,0 von 0,01 bis 0,05% Fluorid-Antikariesmittel und Wasser als den Rest enthält, wobei sich alle Prozentangaben auf das Gewicht beziehen.

12. Zahnpastezusammensetzung mit antimirkobieller Wirkung gegen Plaque-bildende Mikroorganismen und dadurch gekennzeichnet, daß sie eine wirksame Menge von 0,5 bis 2,0% eines oder mehrerer hochreiner Alkalimetallsalze(s) von Dodecylsulfat mit einem Gehalt von weniger als 5% an nicht-Dodecyl Alkylsulfatsalz(en), welche(s) in einem oder mehreren physiologisch mit menschlichen Zähnen und Mundgeweben verträglichen Träger(n) enthalten ist (sind), einen physiologisch akzeptablen sauren Puffer zur Aufrechterhaltung des pH Wertes im Bereich von 3,0 bis 5,0, von 0,5 bis 2,0% eines Alkalimetall-N-acylsarcosinats, von 25 bis 40% Dentalschleifmittelpartikel, von 1 bis 4% Verdickungsmittel und von 20 bis 40% Feuchthaltemittel umfaßt, daß der Rest aus Wasser besteht, und daß sich alle Prozentangaben auf das Gewicht beziehen.

13. Zahnpastezusammensetzung nach Anspruch 12, dadurch gekennzeichnet, daß sie auch ein Fluorid-Anitkariesmittel enthält.

14. Zahnpastezusammensetzung nach Anspruch 13, dadurch gekennzeichnet, daß das Fluorid Natriummonofluorphosphat ist.

15. Zusammensetzung nach irgendeinem der vorhergehenden Ansprüche, dadurch gekennzeichnet, daß das Sarcosinat N-lauroylsarcosinat ist.

16. Zusammensetzung nach Anspruch 15, dadurch gekennzeichnet, daß das Sarcosinat Natrium-N-lauroylsarcosinat ist.

17. Zahnpastezusammensetzung nach Anspruch 12, dadurch gekennzeichnet, daß sie von 0,5 bis 2,0% hochreines Natriumdodecylsulfat, von 25 bis 40% Dentalschleifmittelpartikel, von 1 bis 4% Verdickungsmittel, von 0,5 bis 3,0% physiologisch akzeptables Pigment, von 0,5 bis 2,0% Natrium-N-lauroylsarcosinat, von 20 bis 40% Feuchthaltemittel, von 0,1 bis 0,3% Süßstoff, von 0,1 bis 1% Fluorid-Antikariesmittel, von 1,0 bis 5,0% Phosphatpuffer, ausreichend Phosphorsäure, um den pH Wert der Zusammensetzung auf etwa pH 5,0 einzustellen, etwa 0,5% ätherisches Öl als Geschmacksstoff umfaßt, daß der Rest Wasser ist, und daß all Prozentangaben sich auf das Gewicht beziehen.

**Patentansprüche für den Vertragsstaat: AT**

1. Verfahren zur Herstellung einer dentalhygienischen Zusammensetzung mit antimikrobieller Wirkung gegen Plaque-bildende Mikroorganismen, welches Verfahren dadurch gekennzeichnet ist, daß es die Inkorporierung einer wirksamen Menge von 0,1 bis 2,0 Gew.-%, bezogen auf das Gewicht der Zusammensetzung, eines oder mehrerer besonders reinen(r) Alkalimetallsalze(s) von Dodecylsulfat mit einem Gehalt von weniger als 5% an nicht-Dodecyl Alkylsulfatsalz(en), 0,5 bis 2,0 Gew.-%, bezogen auf das Gewicht der Zusammensetzung, eines Alkalimetall-N-acylsarcosinats und eines physiologisch akzeptablen sauren Puffers zur Aufrechterhaltung des pH Wertes der Zusammensetzung im Bereich von 3,0 bis 5,0, in einen oder mehrere mit menschlichen Zähnen und Mundgeweben verträgliche(n) Träger umfaßt.

2. Verfahren nach Anspruch 1, dadurch gekennzeichnet, daß der Anteil an nicht-Dodecyl Alkylsulfatsalz(en) weniger als 2% ist.

3. Verfahren nach Anspruch 1, dadurch gekennzeichnet, daß der Anteil an nicht-Dodecyl Alkylsulfatsalz(en) weniger als 1% beträgt.

4. Verfahren nach irgendeinem der vorhergehenden Ansprüche, dadurch gekennzeichnet, daß der Träger Wasser umfaßt, und der pH Wert der Zusammensetzung etwa 4, 5 ist.

5. Verfahren nach irgendeinem der Ansprüche 1 bis 4, dadurch gekennzeichnet, daß die Zusammensetzung von 0,5 bis 2,0 Gew.-% des hochreinen Alkalimetallsalzes von Dodecylsulfat enthält.

6. Verfahren zur Herstellung einer flüssigen Mundwasserzusammensetzung mit antimikrobieller Wirkung gegen Plaque-bildende Mikroorganismen, welches Verfahren dadurch gekennzeichnet ist, daß es die Inkorporierung von 0,1 bis 2,0 Gew.-% eines oder mehrerer hochreinen(r) Alkalimetallsalze(s) von Dodecylsulfat mit einem Gehalt an nicht-Dodecyl Alkylsulfatsalz(en) von weniger als 5%, 0,5 bis 2,0 Gew.-% eines Alkalimetall-N-acylsarcosinats und eines physiologisch akzeptablen sauren Puffers zur Aufrechterhaltung des pH Wertes der Zusammensetzung im Bereich von 3,0 bis 5,0, welche in einem oder mehreren mit menschlichen Zähnen und Mundgeweben physiologisch verträglichen Träger(n) gelöst sind, und von 10 bis 30 Gew.-% Äthylalkohol in eine geeignete Basiszusammensetzung umfaßt, wobei sich die Prozentangaben auf das Gewicht der Endzusammensetzung beziehen.

7. Verfahren nach Anspruch 6, dadurch gekennzeichnet, daß die Zusammensetzung von 0,1 bis 0,5 Gew.-% des hochreinen Alkalimetallsalzes von Dodecylsulfat enthält.

8. Verfahren nach Anspruch 6 oder 7, dadurch gekennzeichnet, daß die Zusammensetzung Wasser oder einen anderen wässrigen Träger enthält und auch von 0,2 bis 3,0 Gew.-% ätherisches Öl als Geschmacksstoff und von 0,1 bis 0,2 Gew.-% nicht-ionische(s) oberflächenaktive(s) Mittel als Dispergierungsmittel dafür enthält.

9. Verfahren nach Anspruch 6, 7 oder 8, dadurch gekennzeichnet, daß die Zusammensetzung einen Wassergehalt von 50 bis 90 Gew.-% hat und bis zu 20 Gew.-% Feuchthaltemittel enthält.

10. Verfahren nach irgendeinem der Ansprüche 6 bis 9, dadurch gekennzeichnet, daß die Zusammensetzung von 0,01 bis 0,05 Gew.-% in Wasser oder einem anderen wässrigen Medium als Träger gelöstes Zinkchlorid enthält.

11. Verfahren nach Anspruch 6, dadurch gekennzeichnet, daß es die Inkorporierung von 0,1 bis 0,5% hochreinem Natriumdodecylsulfat, von 0,5 bis 2,0 Gew.-% eines Alkalimetall-N-acylsarcosinats, von 10 bis 30% Äthylalkohol, von 0,01 bis 0,05% Zinkchlorid, von 0 bis 20% Feuchthaltemittel, von 0,2 bis 3,0 ätherischem Öl als Geschmacksstoff, von 0,1 bis 0,2 nichtionischem Dispergierungsmittel, von 0,002 bis 0,005% Farbstoff, einer ausreichenden Menge an physiologisch akzeptabler Säure, um den pH Wert der Zusammensetzung auf einen pH Wert im Bereich von 3,0 bis 5,0 zu bringen, Pufferlösung zur Aufrechterhaltung des pH Wertes der Zusammensetzung bei einem pH Wert im Bereich von 3,0 bis 5,0 und von 0,01 bis 0,05% Fluorid-Antikariesmittel in Wasser umfaßt, wobei alle Prozentangaben sich auf das Gewicht und auf die Zusammensetzung beziehen.

12. Verfahren zur Herstellung einer Zahnpastezusammensetzung mit antimikrobieller Wirkung gegen Plaquebildende Mikroorganismen, welches dadurch gekennzeichnet ist, daß eine wirksame Menge von 0,5 bis 2,0% eines oder mehrerer hochreinen(r) Alkalimetallsalze(s) von Dodecylsulfat mit einem Gehalt von weniger als 5% an nicht-Dodecyl Alkylsulfatsalz(en), welche in einem oder mehreren mit menschlichen Zähnen und Mundgeweben physiologisch verträgenlichen Träger(n) gelöst sind, einem physiologisch akzeptablen sauren Puffer zur Aufrechterhaltung des pH Wertes im Bereich von 3,0 bis 5,0, von 0,5 bis 2,0% eines Alkalimetall-N-acylsarcosinats, von 25 bis 40% Dentalschleifmittelpartikel, von 1 bis 4% Verdickungsmittel und von 20 bis 40% Feuchthaltemittel in Wasser inkorporiert werden, wobei alle Prozentangaben sich auf das Gewicht und auf die Zusammensetzung beziehen.

13. Verfahren nach Anspruch 12, dadurch gekennzeichnet, daß in die Zusammensetzung ein Fluorid-Antikariesmittel inkorporiert wird.

14. Verfahren nach Anspruch 13, dadurch gekennzeichnet, daß das Fluorid Natriummonofluorphosphat ist.

15. Verfahren nach irgendeinem der vorhergehenden Ansprüche dadurch gekennzeichnet, daß das Sarcosinat N-Lauroylsarcosinat ist.

16. Verfahren nach Anspruch 15, dadurch gekennzeichnet, daß das Sarcosinat Natrium-N-lauroylsarcosinat ist.

17. Verfahren zur Herstellung einer Zahnpastezusammensetzung nach Anspruch 12, dadurch gekennzeichnet, daß von 0,5 bis 2,0% hochreines Natriumdodecylsulfat, von 25 bis 40% Dentalschleifmittelpartikel, von 1 bis 4% Verdickungsmittel, von 0,5 bis 3,0% physiologisch akzeptables Pigment, von 0,5 bis 2,0% Natrium-N-lauroylsarcosinat, von 20 bis 40% Feuchthaltemittel, von 0,1 bis 0,3% Süßstoff, von 0,5 bis 1% Fluorid-Antikariesmittel, von 1,0 bis 5,0% Phosphatpuffer, ausreichend Phosphorsäure, um den pH Wert der Zusammensetzung auf etwa 5,0 einzustellen, und etwa 0,5% ätherisches Öl als Geschmacksstoff in Wasser inkorporiert werden, wobei alle Prozentangaben sich auf das Gewicht und auf die Zusammensetzung beziehen.

**Revendications pour les Etats Contractants: BE, CH, DE, FR, GB, IT, LI, LU, NL, SE**

1. Une composition d'hygiène dentaire présentant une activité anti-microbienne vis-à-vis des micro-organismes formateurs de la plaque dentaire et comprenant une quantité efficace de 0,1 à 2,0% en poids, exprimés par rapport au poids de la composition, de un ou plusieurs dodécylsulfates de métaux alcalins à pureté élevée présentant une teneur de moins de 5% en alkylsulfate(s) non dodécylique(s), de 0,5 à 2,0% en poids, exprimés par rapport au poids de la composition, d'un N-acylsarcosinate de métal alcalin et contenant un tampon acide physiologiquement acceptable pour maintenir le pH de la composition dans une valeur comprise entre 3,0 et 5,0, dissous dans un ou plusieurs véhicules physiologiquement compatibles avec les tissus buccaux et la dentition humaine.

2. Une composition ainsi que revendiquée dans la revendication 1, dans laquelle ladite teneur en alkylsulfate(s) non dodécylique(s) est inférieure à 2%.

3. Une composition ainsi que revendiquée dans la revendication 1, dans laquelle ladite teneur en alkylsulfate(s) non dodécylique(s) est inférieure à 1%.

4. Une composition ainsi que revendiquée dans l'une quelconque des revendications précédentes, dans laquelle le véhicule est formé d'eau et le pH de la composition est de l'ordre de 4, 5.

5. Une composition ainsi que revendiquée dans l'une quelconque des revendications 1 à 4, qui contient de 0,5 à 2,0% en poids dudit dodécylsulfate de métal alcalin à pureté élevée.

6. Une composition de bain de bouche liquide présentant une activité anti-microbienne vis-à-vis des micro-organismes formateurs de la plaque dentaire et comprenant de 0,1 à 2,0% de un ou plusieurs dodécylsulfate(s) de métaux alcalins à pureté élevée, présentant une teneur inférieure à 5% en alkylsulfate(s) non dodécylique(s), de 0,5 à 2,0% en poids de N-acylsarcosinate de métal alcalin et contenant un tampon acide physiologiquement acceptable afin de maintenir le pH de la composition dans une valeur comprise entre 3,0 et 5,0, dissous dans un ou plusieurs véhicules compatibles du point de vue physiologique avec les tissus buccaux et la dentition humaine et de 10 à 30% en poids d'alcool éthylique, ces pourcentages étant exprimés par rapport au poids de la composition.

7. Une composition de bain de bouche liquide ainsi que revendiquée dans la revendication 6, qui contient de 0,1 à 0,5% en poids de dodécylsulfate d'un métal à pureté élevée.

8. Une composition de bain de bouche liquide ainsi que revendiquée dans la revendication 6 ou 7, qui contient de l'eau ou un autre véhicule aqueux et contient également de 0,2 à 3,0% en poids d'un parfum consistant en une huile essentielle et de 0,1 à 0,2% en poids d'agent tensio-actif(s) non ionique(s) agissant en tant que dispersant.

9. Une composition de bain de bouche liquide ainsi que revendiquée dans les revendications 6, 7 ou 8, qui présente une teneur en eau de 50 à 90% en poids et contient jusqu'à 20% en poids d'un humectant.

10. Une composition de bain de bouche liquide ainsi que revendiquée dans l'une quelconque des revendication de 6 à 9, qui contient également de 0,01 à 0,05% en poids de chlorure de zinc dissous dans de l'eau ou un autre milieu aqueux agissant en tant que véhicule.

11. Une composition de bain de bouche liquide ainsi que revendiquée dans la revendication 6 et comprenant de 0,1 à 0,5% de dodécylsulfate de sodium à pureté élevée, de 0,5 à 2,0% en poids de N-acylsarcosinate de métal alcalin, de 10 à 30% d'alcool éthylique, de 0,01 à 0,05% de chlorure de zinc, de 0 à 20% d'un humectant, de 0,2 à 3,0% d'un parfum à base d'une huile essentielle, de 0,1 à 2,0% d'un agent de dispersion non ionique, de 0,002 à 0,005% de pigment, un acide acceptable du point de vue physiologique en une quantité suffisante pour porter le pH de la composition à un pH de l'ordre de 3,0 à 5,0, une solution tampon pour maintenir le pH de la composition à un pH compris entre 3,0 et 5,0, de 0,01 à 0,05% d'agent anti-carie consistant en fluorure et le reste étant de l'eau, tous le pourcentages étant exprimés en poids.

12. Une composition de pâte dentrifice présentant une activité anti-microbienne vis-à-vis des micro-organismes formateurs de la plaque dentaire de comprenant une quantité efficace de 0,5 à 2,0% en poids de un ou plusieurs dodécylsulfates de métal alcalin à pureté élevée présentant une teneur inférieure à 5% en alkyl-sulfate(s) non dodécylique(s) dissous dans un ou plusieurs véhicules physiologiquement compatibles avec les tissus buccaux et la dentition humaine, un tampon acide physiologiquement acceptable pour maintenir le pH dans une fourchette comprise entre 3,0 et 5,0, de 0,5 à 2,0% d'un N-acrylsarcosinate de métal alcalin, de 25 à 40% d'un abrasif dentaire particulaire, de 1 à 4% d'épaississant et de 20 à 40% d'umectant, le reste étant formé d'eau et tous les pourcentages étant exprimés en poids.

13. Une composition de pâte dentrifice ainsi que revendiquée dans la revendication 12 qui contient également un agent anti-carie à base de fluorure.

14. Une composition de pâte dentrifice ainsi que revendiquée dans la revendication 13, dans laquelle le fluorure consiste en monofluorophosphate de sodium.

15. Une composition de pâte dentrifice ainsi que revendiquée dans l'une quelconque des revendications précédentes, dans laquelle ledit sarcosinate est le N-laurylsarcosinate.

16. Une composition de pâte dentifrice ainsi que revendiquée dans la revendication 15, dans laquelle le sarcosinate et le N-laurylsarcosinate de sodium.

17. Une composition de pâte dentrifice ainsi que revendiquée dans la revendication 12, comprenant de 0,5 à 2,0% de dodécylsulfate de sodium de pureté élevée, de 25 à 40% d'un abrasif dentaire particulaire, de 1 à 4% d'épaississant, de 0,5 à 3,0% d'un pigment acceptable du point physiologique, de 0,5 à 2,0% de N-laurylsarcosinate de sodium, de 20 à 40 d'humectant, de 0,1 à 0,3% d'édulcorant, de 0,1 à 1% d'agent anti-carie à base de fluorure, de 1,0 à 1% de tampon phosphate, suffisamment d'acide phosphorique pour que le pH de la composition soit régle à une valeur d'environ 5,0, environ 0,5% de parfum à base d'huile essentielle, le reste étant de l'eau, tous le pourcentages étant exprimés en poids.

**Revendications pour l'Etat Contractant: AT**

1. Un procédé de production d'une composition d'hygiène dentaire présentant une activité anti-microbienne vis-à-vis des micro-organismes formateurs de la plaque dentaire, ce procédé comprenant l'incorporation, dans un ou plusieurs véhicules physiologiquement compatibles avec les tissus buccaux et la dentition humaine, d'une quantité efficace de 0,1 à 2,0% en poids, exprimés par rapport au poids de la composition, de un ou plusieurs dodécylsulfates de métal alcalin à pureté élevée présentant une teneur inférieure à 5% en alkylsulfates non dodécyliques, de 0,5 à 2,0 en poids, exprimés par rapport de la composition d'un N-acylsarcosinate de métal alcalin et un tampon acide physiologiquement acceptable pour maintenir le pH de la composition dans une valeur comprise entre 3,0 et 5,0.

2. Un procédé selon la revendication 1, dans laquelle ladite teneur en alkylsulfate(s) non dodé-cylique(s) est inférieure à 2%.

3. Un procédé selon la revendication 1, dans laquelle ladite teneur en alkylsulfate(s) non dodé-cylique(s) est inférieure à 1%.

4. Un procédé selon l'une quelconque des revendications précédentes dans laquelle le véhicule est formé d'eau et le pH de la composition est d'environ 4,5.

5. Un procédé selon l'une quelconque des revendications 1 à 4, dans laquelle la composition contient de 0,5 à 2,0% en poids de dodécylsulfate de métal alcalin à pureté élevée.

6. Un procédé pour produire une composition de bain de bouche liquide présentant une activité anti-microbienne vis-à-vis des microorganismes formateurs de la plaque dentaire, ce procédé comprenant l'incorporation dans une composition de base convenable de 0,1 à 2,0% en poids de un ou plusieurs dodécylsulfates de métaux alcalins à pureté élevée, présentant une teneur inférieure à 5% en alkylsulfate(s) non dodé-cylique(s), de 0,5 à 2,0% en poids d'un N-acylsarcosinate de métal alcalin et un tampon acide physiologiquement acceptable pour maintenir le pH de la composition dans une valeur comprise entre 3,0 et 5,0, dissous dans un ou plusieurs véhicules compatibles du point de vue physiologiques avec les tissus buccaux et la dentition humaine et comprenant 10 à 30% en poids d'alcool éthylique, les pourcentages étant exprimés par rapport au poids de la composition.

7. Un procédé selon la revendication 6, qui contient de 0,1 à 0,5% en poids de dodécylsulfate d'un métal alcalin à pureté élevée.

8. Un procédé selon la revendication 6 ou 7, dans laquelle la composition contient contient de l'eau ou un autre véhicule aqueux et contient également de 0,2 à 3,0% en poids d'un parfum consistant en une huile essentielle et de 0,1 à 0,2% en poids d'agent(s) tensio-actif(s) non ionique(s) agissant en tant que dispersant.

9. Un procédé selon la revendication 6, 7 ou 8, dans laquelle la composition présenté une teneur en eau comprise entre 50 à 90% en poids et contient jusqu'à 20% en poids d'un humectant.

10. Un procédé selon une quelconque des revendication 6 à 9, dans laquelle la composition contient de 0,01 à 0,05% en poids de chlorure de zinc dissous dans de l'eau ou un autre milieu aqueux agissant en tant que véhicule.

11. Un procédé selon la revendication 6, qui comprend l'incorporation dans de l'eau de 0,1 à 0,5% de dodécylsulfate de sodium à pureté élevée, de 0,5 à 2,0% en poids de N-acylsarcosinate de métal alcalin, de 10 à 30% d'alcool éthylique, de 0,01 à 0,05% de chlorure de zinc, de 0 à 20% d'un humectant, de 0,2 à 3,0% d'un parfum à base d'un huile essentielle, de 0,1 à 2,0% d'agent de dispersion non ionique, de 0,002 à 0,005% de pigment, un acide acceptable du point de vue physiologique en une quantité suffisante pour porter le pH de la composition à une valeur comprise entre 3,0 et 5,0, une solution tampon pour maintenir le pH de la composition à une valeur comprise entre 3,0 et 5,0, de 0,01 à 0,05% d'agent anti-carie consistant en fluorure, tous le pourcentages étant exprimés en poids et étant exprimés par rapport à ladite composition.

12. Un procédé de production d'une composition de pâte dentifrice présentant une activité anti-microbienne vis-à-vis des microorganismes formateurs de la plaque dentaire qui consiste à incorporer dans de l'eau une quantité efficace de l'ordre de 0,5 à 2,0% du un ou plusieurs dodécylsulfate(s) de métal alcalin à pureté élevée présentant une teneur inférieure à 5% en alkyl-sulfate(s) non dodécylique(s) dissous dans un ou plusieurs véhicules physiologiquement compatibles avec les tissus buccaux et la dentition humaine, un tampon acide physiologiquement acceptable pour maintenir le pH à une comprise entre 3,0 et 5,0, de 0,5 à 2% d'un N-acylsarcosinate de métal alcalin, de 25 à 40% d'un abrasif dentaire particulaire, de 1 à 4% d'épaississant et de 20 à 40% d'humectant, tous les pourcentages étant exprimés en poids et par rapport à ladite composition.

13. Un procédé selon la revendication 12 qui comprend également l'incorporation dans une, composition d'un agent anti-carie à base de fluorure.

14. Un procédé selon la revendication 13, dans la revendication 13, dans laquelle le fluorure consiste en monofluorophosphate de sodium.

15. Un procédé selon une quelconque des revendications précédentes, dans laquelle ledit sarcosinate est le N-laurylsarcosinate.

16. Un procédé selon la revendication 15, dans laquelle le sarcosinate est le N-laurylsarcosinate de sodium.

17. Un procédé de production d'une composition de pâte dentrifice conforme à la revendication 12, qui consiste à incorporer dans de l'eau de 0,5 à 2,0% de dodécylsulfate de sodium de pureté élevée, de 25 à 40% d'un abrasif dentaire particulaire, de 1 à 4% d'épaississant, de 0,5 à 3,0% de pigment acceptable du point physiologique, de 0,5 à 2,0% de N-laurylsarcosinate de sodium, de 20 à 40% d'humectant, de 0,1 à 0,4% d'édulcorant, de 0,5 à 1% d'agent anti-carie à base de fluorure, de 1,0 à 5,0% de tampon phosphate, suffisamment d'acide phosphorique pour régler le pH de la composition à environ 5,0, et environ 0,5% de parfum à base d'huile essentielle, tous le pourcentages étant exprimés en poids et par rapport à la composition.